Europäisches Patentamt

European Patent Office

Office européen des brevets

(11) Publication number: **0 059 563**
**B1**

# EUROPEAN PATENT SPECIFICATION

(45) Date of publication of patent specification: **21.01.87**

(51) Int. Cl.⁴: **A 61 K 39/35,** A 61 K 37/02, A 61 K 35/22

(21) Application number: **82300841.2**

(22) Date of filing: **18.02.82**

(54) An antigen drug and process of producing it for therapy of nonspecific allergic diseases.

(30) Priority: **04.03.81 JP 29941/81**

(43) Date of publication of application:
**08.09.82 Bulletin 82/36**

(45) Publication of the grant of the patent:
**21.01.87 Bulletin 87/04**

(84) Designated Contracting States:
**AT BE CH DE FR GB IT LI NL SE**

(56) References cited:
**DE-A-2 544 183**
**DE-B-1 617 806**
**FR-A-2 218 090**
**FR-M- 6 172**
**GB-A-1 174 974**
**GB-A-2 016 477**
**US-A-3 248 295**
**US-A-4 169 139**
**US-A-4 178 285**

(73) Proprietor: **Kokubu, Tadao**
**2-3, Ooi 2-chome**
**Shinagawa-ku (JP)**

(72) Inventor: **Kokubu, Tadao**
**2-3, Ooi 2-chome**
**Shinagawa-ku (JP)**

(74) Representative: **Davies, Arthur Raymond et al**
**27, Imperial Square**
**Cheltenham, GL50 1RQ (GB)**

Courier Press, Leamington Spa, England.

## Description

This invention relates to an antigen drug and a process for producing it and making it available for oral, percutaneous and permucous membrane immunotherapy through autoimmunization for the syndrome identical with "feeling cold" in English (so called "Hiesho" in Japanese), i.e. contracting non-specific allergic diseases caused from auto-antigen-antibody reaction.

Until recently such non-specific allergic diseases have never been differentiated exactly from specific allergic diseases, although such diseases have been studied since ancient times. In the literature the term "non-specific allergy" is often used without discrimination. In my opinion, non-specific allergic diseases are defined by the fact that their onset is due to the action of an antigen (not exactly an exogenous antigen, but an exogenous antigen modified in the body, for example modified immunoglobulin G) or essentially an antigen produced in the body and an antibody or antigen-antibody complexes in co-operation.

From this point of view non-specific allergic diseases resemble autoimmune diseases. In fact both are essentially identical.

Thus autoimmune diseases and non-specific allergic diseases occur at any time without any contact with exogenous antigens but due to endogenous antigen-antibody reaction. These mechanisms are triggered anywhere in the tissues so that, if it occurs in the abdomen, especially the pelvis, we call it "Hiesho" or "feeling cold" and, if it occurs elsewhere we call it the equivalent of "Hiesho".

Non-specific allergic diseases have a high rate of incidence and are capable of causing chronic relapses, especially in women.

These characteristics and symptoms have been confirmed from substantial study results.

1) Non-specific allergic diseases based on an hereditary allergic constitution have their onset when the autonomic nervous system, the internal secretion system and the metabolism receive exogenous or endogenous stimuli and develop various symptoms.

2) Non-specific allergic diseases based on auto-antigen-antibody reaction are amongst various allergic diseases.

3) Non-specific allergic diseases are chronic recurrent diseases and occur at all ages, and especially in women. Non-specific allergic diseases crucially influence menstruation, pregnancy and erotogenic function and thus disturb family life.

4) These non-specific allergic diseases are classified according as to whether they occur in the abdomen, the small pelvis, the wind pipe, the skin, the joints, etc. There are many cases in which the abdomen and small pelvis types are accompanied by blood circulation troubles, and these types are referred to as causing "the feeling of cold" in the narrow sense. Other types are referred to as "allergic equivalent".

5) Non-specific allergic diseases have various combinations of reactions, but never lead to death by themselves.

6) We can classify these diseases as being of types having short and long term duration. The former have a duration of typically a few days and the latter have a duration of typically a few years. The above terms consist respectively of the prodrome, the paroxysm, the disappearance and the panse.

7) Frequent paroxysms of these non-specific allergic diseases leave a physical stigma.

Experimental background:

1) The distinctive erythrocyte agglutination response was recognized when serum of a post partum woman was added to auto-erythrocyte pretreated with tannic acid (diluted 400-fold in distilled water) and also with auto-placental tissue extract of a pregnant woman suffering from non-specific allergic disorders essentially, as well as severe pregnancy intoxication.

2) The γ-globulin fraction obtained by treating multivalent human urine with ammonium sulphate sensitizes rabbits to produce antihuman urine γ-globulin rabbit antiserum. P. L. Mollison modified the original Coombs method to carry out the Coombs direct method. As a result, a positive Coombs response was found to occur in patients who had paroxysms at high rate as compared with normal persons. (In this specification, multivalent human urine is defined as "urine which is collected from a plurality of healthy persons.")

3) It has turned out that anti-human urine γ-globulin rabbit antiserum shows a cross-immune response in various ways to human serum, secretion, excretion, organ and tissue extract.

From the above description, I cannot help concluding that typical antibodies exist broadly within the human body, and have a close relation to the true cause of non-specific allergic diseases.

From ancient times, the use of oral and local therapy using human urine has been widespread as a personal therapy whose pharmaceutical action was unknown. Currently, sugar, which is produced by eliminating protein from human urine, is used percutaneously in desensitization to asthma and other relative allergic diseases with remarkable success.

It was not conventionally assumed that protein derived from blood, except for small quantities of albumin and Bence Jones protein, was excreted in normal human urine. More recently, as analytical methods have made greater progress, it has been found that some kinds of globulin, other than albumin, mucoprotein and glycoprotein, are excreted in human urine, and, in addition, that degraded transferrin, macroglobulin, lipoprotein and so forth exist in normal human urine.

# 0 059 563

The total amount of protein in normal early-morning human urine ranges from 3 mg/dl to 100 mg/dl of which the amount of γ-globulin is about 70%. Most of these excreted proteins are of the glycoprotein type.

As for oral immunity, 40 years ago the Frenchman Besredka studied local immunity against intestinal tract infection and gave pills of heat denatured sterile vaccine. However his trial failed because a large number of blood antibodies were produced in comparison with the extent of local immunity. Then, the creation of local immunity was attempted by giving a mixture of B.C.G. vaccine with milk to babies for intestinal tuberculosis. However B.C.G. reaches the lymph nodes of the mesenteric wall through the intestinal tract and grows slowly and produces a small amount of local immunity but, at the same time, produces a large amount of antibodies systemically. It is well known that Sabin established the antipolio immunity method in which an infant takes poliovirus vaccine internally and acquires antipolio immunity. The mechanism can be explained as follows. In the intestinal wall, weakly toxic poliovirus, S-immuno γ-glubulin A, J-chain and the like produce an immune complex. This immune complex develops into a barrier. The resulting barrier immune complex prevents virus from entering the intestinal tract and further prevents viremia from developing. Generally, S-immuno-globulin A complex plays the role of the barrier against hetero- or iso-protein passing through villi and prevents hetero- or iso-protein from entering the intestinal tract. Therefore, when the amount of S-immunoglobulin A complex is reduced for some reason, the general and autoimmune mechanisms are broken down. As a result, bovine serum albumin antibody increases in amount in the body fluid after drinking cow's milk, but generally heteroprotein is transformed into aminoacid in the intestinal wall and is assimilated by liver enzyme action, and is then reconstructed into autoprotein after being conveyed to the cell.

Except for a specific case, there is no instance in which diet-hetero-protein disturbs the immune mechanism. On the contrary, in the case where iso- or autourea-protein is taken internally, it is clear that it has an influence on the process for producing immunity. There is a response similar to the delayed type cellular immunity as already described above. If iso-protein is transformed into amino acid on the intestinal wall, and causes destruction of the antigen critical group, it does not provoke an immune response and therefore the above description does not hold.

In the cases of killed vaccine and B.C.G. vaccine established by Besredka in order to produce local immunity in the intestinal tract, and the vaccine established by Sabin, the action of the vaccine on lymph nodes of the intestinal wall stimulates cellular immunity and then systemic antibody production results. With respect to non-specific antigens such as iso- or autourea-protein, I cannot help considering that, in precisely the same way as above, systemic immunity is produced.

British Specification No. 1,174,974 describes an antigen drug which is produced from urine obtained from an allergic patient to which $ZnCl_2$ is added and from which a sediment is extracted and purified with methanol. Such a drug is poisonous so that it cannot be orally administered. Furthermore the drug consists mainly of sugars and peptides, so that it has an antigenic faculty, but is not an immunogen by itself. When injected in the human body, it combines with carrier proteins there and exhibits an immunogenic faculty for the first time. Thus it cannot induce Arthus phenomena by itself. It would also not be effective when it is applied locally for skin and mucous allergic diseases.

U.S. Specification No. 3,248,295 discloses extracts of mammalian urine having anti-allergic and anti-bradykinin properties. In particular a procedure for extracting an active material from urine is described, the active extract exhibiting anti-histamine, anti-acetylcholine, anti-bradykinin and anti-5-hydroxytryptamine properties, as well as preventing the anaphylactic reaction. The urine is acidified to a pH of about 3 or below concentrated in vacuo at below about 70°C to less than about one fifth of the original volume. This concentrate, with pH within the range 1.5—9, is then extracted with about 2 to about 10 volumes (per vol. of concentrate) of a solvent selected from water-immiscible 2—10 carbon aliphatic ethers, and mixtures of said ethers with up to about 90 vol. percent of halogenated lower alkyl (i.e. $C_1$—$C_2$) hydrocarbons. The extraction step may be repeated, the organic solvent extracts combined and evaporated to dryness preferably at reduced pressure at a temperature below 90°C. Alternatively the organic phase of this step is extracted with water. The dry residue or aqueous solution obtained is re-extracted with a solvent selected from the above described group, filtered and concentrated to dryness at reduced pressure. The dry residue containing the active principle is preferably redissolved in one of the group consisting of lower ketones, water, 2—10 carbon aliphatic ethers, and mixtures of the ether(s) with up to 90% of lower alkyl halogenated hydrocarbons.

Therefore, one of the objects of the present invention is to provide an antigen drug for treating non-specific allergic diseases, such as "feeling cold", by induction of autoimmunity.

Another object of the invention is to provide an antigen drug for non-specific allergic disease therapy which consists of glycoprotein extracted from normal human urine as its effective ingredient.

Another object of the invention is to provide an antigen drug and a process for producing its effective agent for oral immune therapy by which autoimmunity to non-specific allergic diseases is induced or the establishment of delayed type cellular immunity is promoted.

A further object of the invention is to provide an antigen drug suitable for oral immune therapy of non-specific allergic diseases which patients can take internally with ease and a process for producing the drug.

A further object of the invention is to provide a powdered antigen drug suitable for oral immune therapy of non-specific allergic diseases.

3

**0 059 563**

A still further object of the invention is to provide an antigen drug effective for percutaneous or permucous membrane immune therapy of non-specific allergic diseases, and a process for producing the drug.

A still further object of the invention is to provide an antigen drug, effective for percutaneous or permucous membrane immune therapy of non-specific allergic diseases, which in liquid or ointment form can easily be sprayed on or applied directly to the skin or mucous membrane, and a process for producing the drug.

A still further object of the invention is to provide an antigen drug for non-specific allergic disease therapy which does not have any toxicity as a medicament or oncogenicity.

According to one aspect of the invention there is provided an antigen drug for non-specific allergic disease therapy, characterised in that the drug comprises a glycoprotein obtained by testing urine obtained from healthy persons in accordance with the sulfo-salicylic acid method to prove that it is protein negative, dialysing the protein negative urine with water in a sterile tube, adding to the dialysed urine sodium hydroxide having a pH of 9 to 10 and then anhydrous calcium chloride in an amount of about 10% w/v, stirring to allow the resulting white precipitate to absorb the glycoprotein in the urine, separating as much as possible of the supernatant fluid from the precipitate, refining the said precipitate by repeated centrifugal washing with water and grinding the resulting calcium glycoprotein compound in a sterile mortar after the sterile drying thereof.

According to another aspect of the invention there is provided a process for producing percutaneous and permucous membrane drugs for treatment of non-specific allergic diseases located in skin and mucous membrane, characterised in that the process comprises dialysing urine obtained from healthy persons as the starting material using undistilled water dialysis for about twenty-four hours and distilled water dialysis for about twenty-four hours, adjusting the concentration of glycoprotein in the dialysed urine to about 1000 mg/dl by blowing air through it, and then diluting the resulting urine with a base in the form of a saline solution, water, alcohol or an ointment at a rate of about 100 g of the base to about 1000 mg of the glycoprotein of the urine.

In order that the invention may be more fully understood, preferred embodiments of the invention will now be described, by way of example, with reference to the accompanying drawing, in which the single figure is a graph showing an average pattern for the transition of immune recovery in the case where an antigen drug according to the invention is applied for oral immune therapy.

This invention uses normal urine from healthy persons as a starting material. Appropriate quantities of sodium hydroxide and anhydrous calcium chloride are added to normal urine and then stirred. The resulting white precipitate absorbs molecules of glycoprotein in the urine. As much as possible of the supernatant liquid is separated from the precipitate. After addition of sterile purified water to the precipitate, centrifugal irrigation washing is performed an appropriate number of times to purify the white precipitate. The calcium glycoprotein compound resulting from dehydrating the wet precipitate is triturated to render it suitable for oral therapy. Alternatively, after dialysing normal urine as the starting material using undistilled water dialysis and distilled water dialysis for about twenty-four hours, the concentration of glycoprotein in the dialysed urine is adjusted to about 1000 mg/dl by blowing air through it, and is diluted in the form of a saline solution, water, alcohol or an ointment at a rate of about 100 g of the base to about 1000 mg of the glycoprotein of the urine base so as to render it suitable for percutaneous and permucous membrane therapy. This antigen drug for non-specific allergic disease therapy consists of glycoprotein as the effective component.

An embodiment of the process for producing of the oral antigen drug itself for the therapy of non-specific allergic diseases in this invention is described in detail as follows.

Urine obtained from healthy persons (age 18—35, preferably male) in the early morning, which is proved protein negative by the sulfosalicylic acid method, is collected and then is dialysed with water in a sterile tube until the quantity amounts to 5 litres in all. Tests using microscopy and various kinds of culture are carried out in a part of the urine to ascertain that there are no bacteria, micro-organisms and true fungi present. Additionally, for caution's sake, the urine is sterilized by pressure filtration in an ultrafilter having meshes of 0.45 µm. The resulting urine is the starting material. This urine which is collected from healthy persons shows a weak-acidity of pH 6.5.

Sodium hydroxide solution, pH 9—10, is added to the urine. Specific refined anhydrous calcium chloride is added to the urine at a rate of 10 w/v% and stirred, resulting in the formation of a white precipitate. The amount of molecules of glycoprotein absorbed by and precipitated with the white precipitate reaches 40—50% of the amount of the urine.

Then, after as much as possible of the supernatant liquid is separated without causing turbulence of the precipitate, the precipitate is divided into sterile centrifugal precipitation tubes and then centrifuged and washed at 2000 rpm for fifteen minutes after adding sterile water. This repeated centrifuging and washing operation which is repeated 15 times removes the urine coloration and makes the precipitate white to the naked eye.

Successively, this wet precipitate is enclosed in sterile filter paper and then dried with drying-air under blast (adjusted to 30°C). As a result, a calcium glycoprotein compound is produced. In the last stage, this compound is triturated in a sterile mortar to render it suitable for use as the oral antigen drug of this invention for nonspecific allergic diseases therapy.

4

The characteristics of the oral antigen drug as above produced and its content of glycoprotein are as follows.

The oral antigen drug of this invention is a light-yellow-white, tasteless and odorless powder and the yield is 4.95 g from the amount of 5 I urine. The oral antigen drug is not soluble in water, alkali liquids, organic solvents, but is dissolved in acid liquids of around pH2. Accordingly, to examine the quantities and properties of the glycoprotein in calcium glycoprotein, the oral antigen drug is dissolved in acid and enclosed in a sterile tube. Then this acid liquid is dialyzed with distilled water for 24 hours. The liquid becomes neutral and calcium chloride is eliminated.

With the protein quantity method of T.C.A. Ponceau using trichloroacetic acid and pigment, the content of protein in the original urine is 8.2 mg/dl and the content of protein in the calcium glycoprotein precipitate supernatant liquid is 2.04 mg/dl. Therefore, in the calcium glycoprotein, about 75% of the protein quantity in the urine is captured.

The total protein quantity of dried calcium glycoprotein comes to 308 mg in quantitative conversion by the T.C.A. Ponceau method, and the protein content therein comes to 62.6 mg per gram of powder. In the oral therapy, dried calcium glycoprotein powder is diluted ten times with lactose. A dose of 0.5 g is internally administered once a day so that administration quantity of protein for a dose is 3.13 mg.

The components and content of the oral antigen drug are as follows;

(a) Sugar: (Carbol-sulfuric acid method) 55 mg/dl.

(b) Content of stiffened lipid, organic compounds (such as urine, uric acid and creatine) and minerals except calcium are respectively shown in the table below. Here the reagent is a solution of pH 6.7 in which 0.1 g calcium glycoprotein is dissolved in 10 ml hydrogen chloride of pH 2 and then dialyzed for twenty four hours.

(unit mg/dl)

| main organic compounds other than lipid | urine ni-trogen (N) | uric acid | creatine | bilirubin | sugar |
|---|---|---|---|---|---|
| | 0.5 | 0 | 0.3 | 0 | 2 |

| content of lipid | β-lipo-protein | choles-terol | neutral fat | phos-phatide | free fatty acid |
|---|---|---|---|---|---|
| | 4 | 2 | 2 | 11 | 0.01 |

| content of main minerals | Na | K | Cl | Ca | P | Mg |
|---|---|---|---|---|---|---|
| | 12 meg/l | 0.6 meg/l | 4 meg/l | 5.2 meg/l | 2.17 mg/dl | 2.17 meg/l |

As the immunological properties of glyco-protein in urine, the protein in urine responds to antihuman serum protein (and its fraction) and anti-rabbit (or -goat) serum. Antihuman urine γ-globulin rabbit antisera respond widely to protein antigen in secretion, exudate, organ, and tissue extracts of human serum. Common immune bodies, which widely exist in human body and glycoprotein, are excreted into urine from serum through the kidney.

The oral immune mechanism of calcium glycoprotein in this invention is now stated. The solution of calcium protein polysaccharide in gastric juice liberates protein polysaccharide, since calcium glycoprotein dissolves in acid solution (pH 2—3.5). Then, glycoprotein dissolved in alkaline intestinal juice is absorbed by the villi. Within the intestinal wall, immune globulins (the domain of γ-globulin) in glycoprotein are captured by local macrophage and leukocyte and therefore antigen determination radical, residual bases in immune globulin (the domain γ-globulin) are exposed. This antigen recognition is transmitted to cells of the lymph system in lymph nodules and then blastization of lymphocyte is triggered. Blastized lymphocytes propagated by fission cause a chain reaction repeatedly in themselves by stimulation of a biological product similar to lympho kine. As a result, the antibody produced by blast cells corrects breaking of immunity in body fluids and keeps immune equilibrium for a long time in cooperation with cellular and body fluid immunities.

The oral immune therapy will now be described.

[I] Internal use application

A dose of 0.5 g calcium glycoprotein powder (containing 3.13 mg protein), which is 10 times as weak as its original through dilution with lactose or glucose, is internally taken once a day on an empty stomach. In the initial immunity, a dose is internally taken at the same time for three days in a row. These three doses for three days are called 1 Kur. The medicine is taken with a little water in the same way as general powder

5

medicines are taken, but it is better to take this medicine with acidulous liquid of a pickled plum, diluted hydrochloric acid limonaze and so forth.

[II] Target of therapy

Careful examination of nonspecific allergic patients is carried out to try to remove mingled symptoms caused from organic diseases. Further, a 5 mg tablet of pledonisolon, which is a type of adreno-cortical hormone drug is taken twice a day, for a week to ascertain disappearance of symptomscomplexes. Immune therapy is done on the basis that symptoms are due to mainly functional disorders. The following report is based on physical examinations, selecting 25 patients (male 5, female 20, age 16—65) at random among 102 patients who received treatment.

[III] Summary of complaints

Patients had been suffering from the following complaints or their combination as started in Table I for two to thirteen years.

TABLE I

| Order | Complaint | % |
|-------|-----------|---|
| 1 | abdominal inflation and pain | 92 |
| 2 | low back pain | 72 |
| 3 | coldness of limbs, abdomen and waist | 68 |
| 4 | anorexia, nausea, emesis | 64 |
| 5 | vertigo, heavy-headedness, orthostatic syncope, cephalalgia | 64 |
| 6 | constipation | 56 |
| 7 | loose passage, diarrhea | 52 |
| 8 | general fatique | 48 |
| 9 | weight loss | 44 |
| 10 | leukorrhea disorder | 40 |
| 11 | dysmenorrhea | 35 |
| 12 | shoulder, back inflamation and pain | 32 |
| 13 | lethargy, sleeplessness | 28 |
| 14 | joint pain, myalgia, neuralgia | 28 |
| 15 | vulvar pruritus | 15 |
| 16 | hemorrhoids | 15 |

In addition to the Complaints as in Table I, eczema, common cold, asthma, urticaria, sterility, purpura and others were variously found.

[IV] Result of treatment and standard of judgment

1. Two months before and after the treatment, the following criteria are comparably examined and then the effect is generally judged.

(1) Alleviation or disappearance of main symptom and complaint, improvement of existing stigma in light of consultation, and weight increase.

(2) Improvement of objective index

The cellular and humoral responses known in immunology were practiced in accordance with necessity.

The above criteria, by the usual immunocellular and -humoral examinations were determined as follows:

6

Materials

Early morning autourine was taken as antigen. Autoblood was taken simultaneously, partially for autoserum separation and partially as heparin blood for cellular elements separation, amounting to about 20 ml in total.

Experiments

1. L.M.T. Here autourine was diluted with physiologic NaCl solution from 1000 to 10 billion fold in 6 steps.

Separated autoleucocytes were incubated with autourine dilution series at 37°C for 1 hour. Thus pretreated leucocytes were quantitatively poured into agarose wells while in the center well leucocytes pretreated with P.B.S. negative solution was poured.

After 24 hours incubation at 37°C, agarose schale was taken out and the leucocytes migration area was examined.

Results

In nonspecific allergic diseases leucocytes migration was extremely depressed due to mingled lymphokines.

2. P.H.A. In this experiment autolymphocytes blasting ratio was compared between 10 million fold diluted autourine, P.H.A. (T cell mitogen), 20 thousand fold diluted and P.B.S. negative contrast after 24 hours incubation at 37°C.

Results

If immunity was kept u>p>(−), immune activity is raised. If immunity was kept p>u>(−), autoallergy is raised.

u: urine, p: P.H.A.

3. T.B.D. cell count. Performed as usual.

Results

In allergic stadium T cell % decrease under 70% while B cell % increase above 20%. In immune phase T cell % above 80% while B cell % under 12%. C cell behavior was not constant.

4. Total lymphocytes number decrease in allergic stadium, while in immune phase it increases.

5. Immuno γ-globulin quantity shows no remarkable changes in any case.

However, in a judgment of the present status and transition of nonspecific allergy which is basically considered as non-specific autoimmune disease, it is difficult to conclude that some responses always reflect immune status faithfully. Accordingly, the four tests described below are practiced as a necessary indication.

① Intracutaneous response using refined urine glycoprotein (containing 10 mg/dl).

② Action of glycoprotein in refined autourine to autoperipheral lymphocytes (with other kinds of leukocytes mixed) causes their blasting, agglutination, and fission (mitogenic function like some vegetable mitogens, for example P.H.A.). Hence, it is inspected opto-microscopically and then compared in a multiple dilution row of glycoprotein in refined autourine.

③ As described above, auto-antibody is absorbed on autoerythrocyte membrane. Agent of refined glycoprotein in autourine to autoerythrocyte pretreated with tannic acid, causes erythrocytes to agglutinate. Polyvalency of autoantibody is judged by comparing multiple dilution of autourine is row; (direct Coombs).

④ Refined autourine glycoprotein produces clot to auto blood plasma under existence of tannic acid solution (400 fold diluted with distilled water). There are individual differences in relation between dilution of glycoprotein in urine and clot time (first step, second step and completion). These differences are corelative to the symptoms of a patient. In the nonspecific allergic diseases group screening especially No. 4 examination relflex well recent, conditions of diseases and autoimmunity disharmony, and further ②—④ shows positive or negative correlation in the same case.

2. Result

The cure rate is shown in the following table on the basis of the above judgments and criteria.

**0 059 563**

(1) Cure Table

| Results | Judgments and criteria | Among 5 men | % | Among 20 women | % | Total |
|---|---|---|---|---|---|---|
| very good | main symptom: disappearance of most of stigma and weight increasing index: greatly improved | 4 | 16 | 17 | 68 | 84% |
| better | main symptom: getting better of most of stigma and weight increasing index: improved | 1 | 4 | 1 | 4 | 8% |
| unchanged | main symptom: unchangeability of stigma, unchangeability of weight index: unchanged | 0 | 0 | 1 | 4 | 4% |
| worse | main symtom: the worse of stigma and weight decreasing index: worse | 0 | 0 | 1 | 4 | 4% |

As shown in the above table, the very good and better cases in results amounted to 92% of all cases.

(2) An unchangeable case

A woman of 45 years had been suffering from non-specific allergic diseases for twenty years and this was accompanied with depression for ten years. The result is shown in the column of the unchanged in the above cure table. This case was judged unchangeable; however, it was found that the condition of the patient got better during the past four months. But the patient took medicine internally after one and half years (booster oral immunization).

(3) A worse case

A woman of 43 years had been suffering from non-specific allergic diseases for ten years and this is accompanied with rheumatoid arthritis for a few years. Two months after she took glycoprotein medicine internally, she had repeatedly a slight fever and reddening, swelling and pain of joints with weight decreasing. The index was worse. This case was in typical allergic disorder. The further treatment was stopped at once.

[V] Transient irritation symptom (allergy symptom) after the internal therapy. Transition to and continuation of immune equilibrium (judgment of the time limit and necessity of re-treatment)

1. It is considered that this therapy method activates systemical cellular immunity (and following humoral immunity) through the intestinal tract. Therefore this method is characterized by appearance of transient allergy symptom within the range of 24—48 hours after contact of glycoprotein with lymph nodes in the mesenteric wall.

2. (1) The situation in which no symptom was complained of. 16% (4 cases)
(2) The situation in which condition and complaint were transiently worse. 52% (13 cases)
(3) The situation in which a hitherto unexperienced symptom appeared. 32% (8 cases)

TABLE of items in (3)

| Symptom | No. | % | Symptom | No. | % |
|---|---|---|---|---|---|
| urticaria | 2 | 25 | asthma | 2 | 25 |
| arthritis, arthralgia | 1 | 12.5 | diarrhea | 1 | 12.5 |
| rhinitis allergic | 1 | 12.5 | purpura | 1 | 12.5 |

8

# 0 059 563

3. The duration of allergic phase from the first day the drug was internally taken

| Duration | No. | % | Duration | No. | % |
|---|---|---|---|---|---|
| 0 day | 4 | 16 | 6—7 days | 2 | 8 |
| 1 day | 1 | 4 | 8—15 days | 1 | 4 |
| 2 days | 4 | 16 | 16 days—1 month | 1 | 4 |
| 3 days | 7 | 28 | 1 month— | 1 | 4 |
| 4—5 days | 4 | 16 | | | |

That is, after glycoprotein was given, 84% out of the total cases had a transient allergic phase. 88% out of the cases, including non-symptom cases, experienced transient allergic symptoms within 7 days after taking the glycoprotein internally.

4. Transition of immune recovery

The transition period in which the immunizing mechanism is accelerated, where patient falls into a transient allergic condition and before long reaches equilibrium, shows extreme individual differences. According to age, sex, the degree of non-specific allergic diseases, location of the main symptom, presence or absence of accompanying diseases, the duration of the oral immunity is modified.

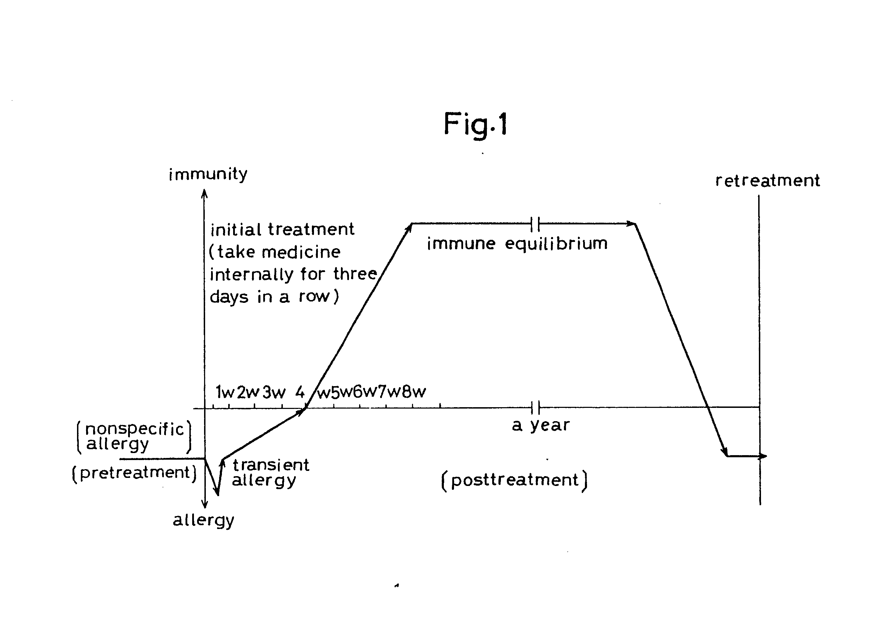

As the average pattern of this transition is shown in Figure 1, the duration for the equilibrium is about two months.

5. Condition posterior to completion of equilibrium

(1) The improvement and absence of subjective symptoms, that is, especially great improvement of various symptoms (feeling of cold, slight fever, heavy-headedness, sleepiness, irritation, shoulder discomfort, abdominal and low back pain) due to autoimmune disturbances and interruption in peripheral blood circulation, improvement of objective stigmata, normalization of stomach function, improvement of metabolism and so forth equally result in weight increasing and recovery of a feeling of health.

(2) There may be unavoidable cases, however, where recovered autoimmunity is disturbed transiently owing to daily and seasonable factors after equilibrium is attained. However, this unbalanced condition is by far more stable than that of pre-treatment and becomes normal in a shorter time. A remarkable symptom is the increase in anti-inflammation faculty. Further it seems to have prophylactic action against malignant tumor eruption, but this cannot be verified.

6. Additional immunity and prognosis

The duration of equilibrium is from one to a few years but is strongly subject to the individual differences and some conditions stated in the above transition of immune recovery. The timing for additional immunity is determined by the reduction of indices and the reappearance of symptoms and complaints. For additional immunity, glycoprotein doses are determined according to said criteria, whether the medicines are taken three times, or the medicines are taken twice and a booster is given a week later, or a booster is only taken once.

Rational repetition of this therapeutic method settles homeostatis of immunity forever without any necessity for re-treatment.

7. Side effects (bad reaction)

(1) Over-sensitization

In the initial immunization, calcium glycoprotein diluted ten times in the amount of 0.5 g (containing protein of 3.13 mg) a day is internally taken 3 times for 3 days in a row. If the medicine is internally taken 7 times for 7 days in a row, the patient would, without exception, develop a crucial allergic condition. The patient would not die but would suffer for a few years. The same case occurs when too much of calcium glycoprotein or protein is internally taken for three days in a row or when retreatment is begun by misjudging the timing of the end of immunity equilibrium.

(2) Remote influences of calcium glycoprotein

Urine calcium glycoprotein need not be considered for its undesirable effect upon oncogenesis and genetic consequences etc. because of its biological origin.

Multivalent human urine as starting material is treated in the same way as in the production of said oral antigen drug. It is dialyzed with undistilled water for about 24 hours and then distilled water for about 24 hours. Successively, the resulting multivalent human urine is dried artificially by air and then the protein

9

# 0 059 563

concentration is adjusted to 1000 mg/dl, and the result is diluted with a base in the form of a saline solution, water, alcohol, an ointment, at the rate of about 100 g of base to about 100 mg of glycoprotein. These antigen drugs are given through a percutaneous and a permucous membrane in chronic allergic diseases of skin; for example, chronic eczema. They show extremely good effect 24—48 hours after use. But, a few days later recidive relapse of these diseases is seen, so that these drugs need to be used repeatedly. Probably, it is assumed that absorbed glycoprotein shows cellular immunity effect under interaction between cells after contact with subcutaneous lymph nodes but doesn't show local immunity for a long time because the subcutaneous lymph organ is a low level one as the immune organ and the resulting immune compound is diluted and dispersed by the circulatory system.

Here, the local use on a percutaneous and a permucous membrane is explained.

(1) Application to chronic skin eczema

If glycoprotein is applied to exudate eczema once a day, dampness is stopped after 24—48 hours and pruriginous effect disappears after drying. The progress is extremely favorable.

(2) Application to seborrheic eczema of the head

As in (1), the itchiness is stopped and the resultant dandruff is reduced.

(3) In urticaria, skin herpes, extremely good effect is shown.
(4) In cases (1) and (2), however, if the local use is stopped for a few days, the symptoms reappeared.

This point is basically different from the results observed for oral immunity and most important. Accordingly, in chronic eczema, the local use is repeated at least every three days and in addition the oral therapy of calcium glycoprotein is used simultaneously.

As described above, various kinds of clinical experiments were carried out on the basis of the immune mechanism of antigen-antibody reaction. As a result, it has become clear that the antigen drug of this invention is extremely effective against non-specific allergic diseases for the treatment of which the conventional drugs do not provide comparable results.

The oral, the percutaneous and the permucous membrane antigen drugs of this invention for therapy of non-specific allergies are especially effective against a feeling of cold from which non-specific allergy patients suffer and against which effective treatment methods have not previously been provided. The production method of this invention makes mass production possible.

**Claims.**

1. An antigen drug for non-specific allergic disease therapy, characterised in that the drug comprises a glycoprotein obtained by testing urine obtained from healthy persons in accordance with the sulfo-salicylic acid method to prove that it is protein negative, dialysing the protein negative urine with water in a sterile tube, adding to the dialysed urine sodium hydroxide having a pH of 9 to 10 and then anhydrous calcium chloride in an amount of about 10% w/v, stirring to allow the resulting white precipitate to absorb the glycoprotein in the urine, separating as much as possible of the supernatant fluid from the precipitate, refining the said precipitate by repeated centrifugal washing with water and grinding the resulting calcium glycoprotein compound in a sterile mortar after the sterile drying thereof.

2. The drug of claim 1, wherein, in said process, the dialysed urine is preserved by freezing, a sample of the urine is examined for the existence of bacteria, micro-organisms and fungi, and, if indicated as necessary by the examination, the urine is sterilised by a pressure filtration process.

3. The drug of claim 1 or 2, wherein, in said process, the quantity of glycoprotein absorbed by said white precipitate reaches 40 to 50% of the quantity of urine.

4. A process for producing percutaneous and permucous membrane drugs for treatment of non-specific allergic diseases located in skin and mucous membrane, characterised in that the process comprises dialysing urine obtained from healthy persons as the starting material using undistilled water dialysis for about twenty-four hours and distilled water dialysis for about twenty-four hours, adjusting the concentration of glycoprotein in the dialysed urine to about 1000 mg/dl by blowing air through it, and then diluting the resulting urine with a base in the form of a saline solution, water, alcohol or an ointment at a rate of about 100 g of the base to about 1000 mg of the glycoprotein of the urine.

**Patentansprüche**

1. Antigenisches Heilmittel für die Behandlung unspezifischer allergischer Krankheiten, dadurch gekennzeichnet, daß das Heilmittel ein Glykoproteid enthält, welches aus dem Harn gesunder Menschen erhalten wird, der nach dem Sulfosalizylsäureverfahren überprüft wurde, um sicherzustellen, daß er Protein-negativ ist, daß der Protein-negative Harn in einem sterilen Rohr mit Wasser dialysiert, dem dialysierten Harn Natriumhydroxid mit einem pH-Wert von 9 bis 10 und dann anhydridisches Kalziumchlorid in einer Menge von etwa 10% w/v zugesetzt und umgerührt wird, um sicherzustellen, daß der erhaltene weiße Niederschlag das im Harn enthaltene Glykoproteid absorbiert, daß soviel als möglich des an der Oberfläche befindlichen Mediums vom Niederschlag abgetrennt, der Niederschlag durch

10

wiederholte Zentrifugierwäsche mit Wasser gereinigt und schließlich die erhaltene Kalzium-Glykoproteid-Verbindung nach ihrer sterilen Trocknung in einem sterilen Mörser zerrieben wird.

2. Heilmittel nach Anspruch 1, dadurch gekennzeichnet, daß bei dem Herstellungsverfahren der dialysierte Harn durch Einfrieren konserviert, eine Probe des Harnes auf das Vorhandensein von Baktieren, Mikroorganismen und Pilzen überprüft und, wenn sich dies aufgrund der Prüfung als notwendig erweist, der Harn durch ein Druck-Filtrationsverfahren sterilisiert wird.

3. Heilmittel nach Anspruch 1 oder 2, dadurch gekennzeichnet, daß bei dem Verfahren die vom weißen Rückstand absorbierte Menge an Glykoproteid 40 bis 50% der Harnmenge ausmacht.

4. Verfahren zur Herstellung percutan und über die Schleimhäute verabreichbarer Heilmittel zur Behandlung unspezifischer allergischer Krankheiten der Haut und Schleimhäute, dadurch gekennzeichnet, daß bei dem Verfahren unter Verwendung von von gesunden Personen erhaltenem Harn als Ausgangsmaterial dieser Harn unter Verwendung von undestilliertem Wasser etwa 24 Stunden dialysiert und dann unter Verwendung von destilliertem Wasser für etwa weitere 24 Stunden dialysiert wird, die Konzentration von Glykoproteid im dialysierten Harn auf etwa 1000 mg/dl durch Hindurchblasen von Luft eingestellt und dann der erhaltene Harn mit einer Grundlage in Form einer Salzlösung, Wasser, Alkohol oder einer Salbe in einem Verhältnis von etwa 100 g der Grundlage auf 1000 mg des Glykoproteids des Harns verdünnt wird.

**Revendications**

1. Médicament antigénique pour le traitement de maladies allergiques non spécifiques, caractérisé en ce que ce médicament comprend une glycoprotéine obtenue en testant, par la méthode à l'acide sulfosalicylique, une urine obtenue de personnes saines pour prouver qu'elle est protéine-négative, en dialysant l'urine protéine-négative avec de l'eau dans un tube stérile, en ajoutant à l'urine dialysée un hydroxyde de sodium ayant un pH de 9 à 10, puis du chlorure de calcium anhydre dans une proportion d'environ 10% p/v, en agitant pour permettre au précipité blanc résultant d'absorber la glycoprotéine contenue dans l'urine, en séparant le maximum possible du liquide surnageant d'avec le précipité, en purifiant ce précipité par lavage centrigue répété à l'eau et en broyant le composé de glycoprotéine calcique résultant dans un mortier stérile, après l'avoir séché dans des conditions stériles.

2. Médicament selon la revendication 1, caractérisé en ce qu'au cours dudit traitement de préparation, l'urine dialysée est conservée par congélation, un échantillon de l'urine est examiné à la recherche de bactéries, de micro-organismes et de champignons et, si cet examen l'indique comme nécessaire, l'urine est stérilisée par un traitement de filtration sous pression.

3. Médicament selon la revendication 1 ou 2, caractérisé en ce qu'au cours dudit traitement de préparation, la quantité de glycoprotéine absorbée par le précipité blanc atteint 40 à 50% de la quantité d'urine.

4. Procédé pour la préparation de médicaments percutanés et permuqueux pour le traitement de maladies allergiques non spécifiques localisées dans la peau et les muqueuses, caractérisé en ce que ce procédé comprend les opérations consistant à dialyser une urine obtenue de personnes saines et servant de matière de départ, en procédant à une dialyse à l'eau non distillée pendant 24 h environ et à une dialyse à l'eau distillée pendant 24 h environ, à régler la concentration de glycoprotéine dans l'urine dialysée à 1000 mg/dl environ par insufflation d'air à travers elle, puis à diluer l'urine résultante avec une base sous forme de solution saline, de l'eau, de l'alcool ou un ouguent, à raison d'environ 100 g de la base pour 1000 mg environ de la glycoprotéine de l'urine.

# Fig.1

immunity

retreatment

initial treatment
(take medicine
internally for three
days in a row)

immune equilibrium

1w 2w 3w  4  w5w6w7w8w

a year

$\left(\begin{array}{l}\text{nonspecific}\\ \text{allergy}\end{array}\right)$

(pretreatment)

transient
allergy

(posttreatment)

allergy

0 059 563